# EUROPEAN PATENT APPLICATION

(11) **EP 3 009 452 A2**
(43) Date of publication of application: **20.04.2016**
(21) Application number: 15190149.3
(22) Date of filing: 16.10.2015
(51) Int. Cl.: C07K 16/28, C07K 16/32, G06T 7/00, A61K 39/00

(54) **IMMUNOTHERAPY USING A LOGICAL "AND" COMBINATION FOR IMMUNE RESPONSE ACTIVATION**

(30) Priority: 17.10.2014 US 201462065582 P; 14.10.2015 US 201514882862
(71) Applicant: Definiens AG, 80636 München (DE)
(72) Inventor: BINNIG, Gerd, 82288 Kottgeisering (DE); SCHMIDT, Günter, 80999 München (DE)
(74) Representative: Winter, Brandl, Fürniss, Hübner, Röss, Kaiser, Polte - Partnerschaft mbB

(57) **Abstract**

A method for treating a malignant tumor in a patient identifies tumor cells using a logical AND operation on antigens on the surfaces of the tumor cells. First and second antigens are determined to be present on the tumor cells. A first medication including a first antibody and a second antibody is administered to the patient. The first antibody is linked to a first dock, and the second antibody is linked to a second dock. In the patient's body, the first antibody binds to a first antigen, and the second antibody binds to a second antigen. After the elapse of a first predetermined interval, a second medication is administered to the patient. The second medication forms a structured binding site when the second medication simultaneously binds to both the first dock and the second dock. After the elapse of a second predetermined interval, a third medication is administered to the patient. The third medication binds only to the structured binding site and activates immune cells of the patient.

## Description

### CROSS REFERENCE TO RELATED APPLICATIONS

This application claims the benefit under 35 U.S.C. §119 of provisional application serial no. 62/065,582, entitled "Immunotherapy Using a Logical "AND" Combination for Immune Response Activation", filed on October 17, 2014. The subject matter of provisional application serial no. 62/065,582 is incorporated herein by reference.

### TECHNICAL FIELD

The present invention relates generally to analyzing target patterns in digital images, and more specifically to a computer-implemented system for detecting and measuring those target patterns.

### BACKGROUND

Cancer is typically diagnosed by analyzing stained samples of tissue from cancer patients and then correlating target patterns in the tissue samples with grading and scoring methods for different kinds of cancers. For example, the malignancy of prostate cancer is indicated by the Gleason grading system, and the severity of breast cancer is diagnosed using the Allred score, the Elston-Ellis score or the HercepTestTM score. And the Fuhrman nuclear grading system indicates the severity of renal cell carcinoma (RCC). Various drug treatments or chemotherapy are administered based on the diagnosis obtained using image analysis. A method is sought, however, of using image analysis to assist with administering drug treatments and to make the drug treatments more effective.

### SUMMARY

A method for treating a malignant tumor in a cancer patient identifies tumor cells using a logical AND operation on antigens on the surfaces of the tumor cells. First and second preselected antigens are determined to be present on the surfaces of tumor cells. A first medication is administered to the patient. The first medication includes a first antibody and a second antibody. The first antibody is linked to a first dock, and the second antibody is linked to a second dock. In the patient's body, the first antibody binds to the first preseleced antigen, and the second antibody binds to the second preselected antigen. For example, the first antibody is anti-HER2, and the first preselected antigen is HER2. For example, the second antibody is anti-PD-L1, which binds to the second preselected antigen, which is the antigen Programmed Death-Ligand 1 (PD-L1). The logical AND therapy uses the PD-L1 antigen together with another antigen to recognize tumor cells. In the patient's body, the first antibody binds to the first antigen HER2, and the second antibody binds to the second antigen PD-L1. PD-L1 is a checkpoint inhibitor that allows cells to escape an immune response. Some types of cancers have acquired the antigen PD-L1 and use it as a tumor escape mechanism. The logical AND therapy uses the PD-L1 antigen together with a second antigen to recognize tumor cells.

After the elapse of a first predetermined interval, a second medication is administered to the patient. The second medication forms a structured binding site when the second medication simultaneously binds to both the first dock and the second dock. The second medication can bind to both of the docks when both the first and second antibodies are bound to first and second antigens that are in close proximity on a tumor cell's surface. After the elapse of a second predetermined interval, a third medication is administered to the patient. The third medication binds only to the structured binding site and activates immune cells of the patient. The immune cells then attack the identified cancer cell.

In another embodiment, a triple-ligand medication is administered in a single-step therapy. Image analysis is first performed on a digital image of a tissue sample of a cancer patient to generate image objects. Then an image analysis system determines that the image objects are cells that have been stained with both a first biomarker and a second biomarker. In a first example, the first biomarker is anti-Programmed Death-Ligand 1 (anti-PD-L1), and the second biomarker is anti-cytokeratin 18 (anti-CK18), and the patient has prostate cancer. In a second example, the first biomarker is anti-Human Epidermal growth factor Receptor 2 (anti-Her2), and the second biomarker is anti-Programmed Death-Ligand 1 (anti-PD-L1), and the patient has breast cancer.

The single-step therapy involves administering an AND molecule to the cancer patient that includes a first antibody and a second antibody. The second antibody has a shape that can bind to its corresponding antigen only when the first antibody is bound to its corresponding antigen. In the first example, the first antibody is anti-PD-L1, and the second antibody is anti-CK18. In the second example, the first antibody is anti-Her2, and the second antibody is anti-PD-L1. The AND molecule includes a binding site that can bind to immune cells only when both the first antibody and the second antibody are bound to their corresponding antigens. The binding site is opened or unfolded by a conformation of the AND molecule induced by the binding of both the first antibody and the second antibody to their corresponding antigens. In one example, the binding site is anti-OX40 that binds to tumor necrosis factor receptor OX40 on immune cells.

Other embodiments and advantages are described in the detailed description below. This summary does not purport to define the invention. The invention is defined by the claims.

### BRIEF DESCRIPTION OF THE DRAWINGS

The accompanying drawings, where like numerals indicate like components, illustrate embodiments of the invention.
FIG. 1 is a diagram of a system for analyzing digital images using both statistical pixel-oriented analysis and object-oriented analysis that links pixels to data objects forming hierarchical networks.
FIG. 2 shows an exemplary hierarchical network that is generated by the image analysis system of FIG. 1.
FIG. 3 shows an AND molecule, a glue molecule and a binding molecule near a cancer cell.
FIG. 4 illustrates a therapy using first, second and third medications that implement a logical AND combination.
FIG. 5 shows a bound configuration of the second medication that permits a structured binding site to be formed when the second medication is bound to the glue docks of both antibodies.
FIG. 6 illustrates another application of the therapy using the first, second and third medications.
FIG. 7 shows a second step of the application of FIG. 6 in which the second medication attaches to the docks of both antibodies.
FIG. 8 shows a third step in which the third medication selectively binds only to structured binding sites formed by the combination of the second medication with the docks of the first medication.
FIG. 9 illustrates a rare occurrence when the third medication attracts immune cells to a cell that does not have both antigens in close proximity on the cell membrane.
FIG. 10 illustrates a therapy in which the first medication is a combination of a first molecule without a glue dock and a second molecule with a glue dock.
FIG. 11 illustrates another therapy in which neither of the molecules of the first medication has a glue dock.
FIG. 12 illustrates a second approach of the novel therapy in which selected immune cells are reprogrammed to perform the selective binding accomplished by the first and second medications.
FIG. 13 shows a first configuration of a novel AND molecule with three ligands according to an embodiment of the invention.
FIG. 14 illustrates a second configuration of the AND molecule of FIG. 13 bound by two antibodies to a tumor cell.
FIG. 15 illustrates a third configuration of the AND molecule of FIG. 13 in which a binding site is opened when both of the antibodies are bound to their corresponding antigens.
FIG. 16 illustrates an immune cell bound to the binding site on the third configuration of the AND molecule of FIG. 13.

### DETAILED DESCRIPTION

Reference will now be made in detail to some embodiments of the invention, examples of which are illustrated in the accompanying drawings.

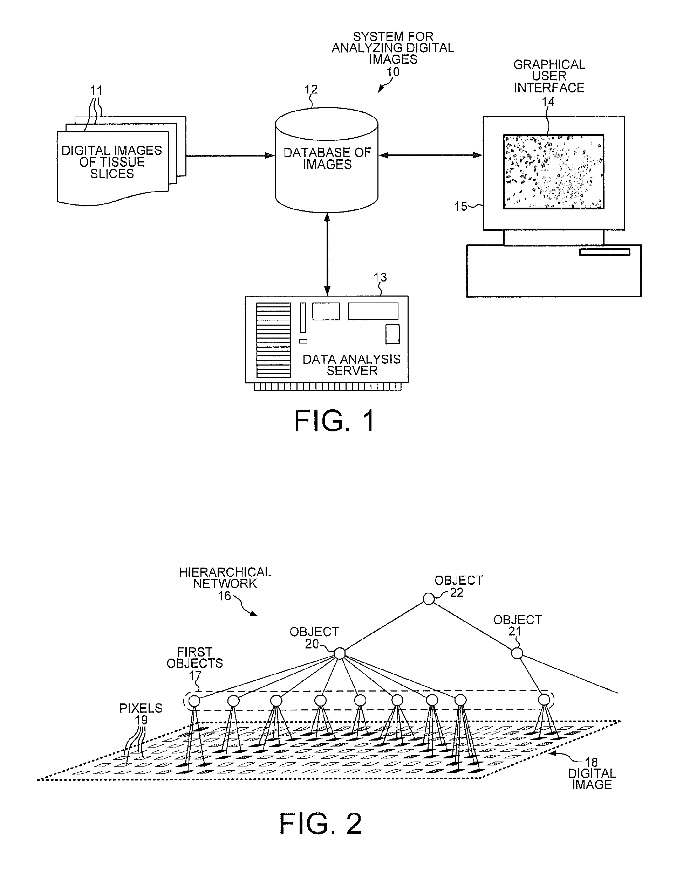

FIG. 1 shows a system 10 for analyzing digital images of tissue samples stained with various biomarkers, such as tissue stained with hematoxylin and eosin (H&E) or tissue stained with a protein-specific antibody using immunohistochemistry (IHC). Digital images 11 of the stained tissue slices are acquired at high magnification. A typical digital image of a tissue slice has a resolution of 100,000 x 200,000 pixels, or 20 billion pixels. The acquired digital images 11 are stored in a database 12 of digital images. Image analysis software executing on a data analysis server 13 then performs intelligent image processing and automated classification and quantification. The image analysis software is a computer program product tangibly embodied on a computer-readable storage medium in server 13 and comprises computer readable and executable program instructions that when executed by a processor on server 13 provide a visual display on a graphical user interface 14 of an interconnected display device 15, such as a personal computer. The image analysis software transforms unlinked input data in the form of pixels into a hierarchical semantic network of objects.

System 10 analyzes, grades, scores and displays the digital images 11 of tissue slices that have been stained with various biomarkers. The image analysis program segments and classifies objects in the digital images 11. The program prepares links between some objects and thereby generates higher hierarchically ranked objects. The image analysis program provides the higher hierarchically ranked objects with properties, classifies them, and then links those objects again at a still higher level to other objects. The higher hierarchically ranked objects are used to find target patterns in the images. More easily detected starting data objects are first found and then used to identify harder-to-find data objects in the hierarchical data structure.

FIG. 2 illustrates exemplary hierarchical network 16 that is generated by image analysis system 10. System 10 generates first objects 17 from a digital image 18 based on the stained tissue. The image analysis program of system 10 uses object-oriented image analysis to generate data objects of hierarchical semantic network 16 by linking selected pixels 19 to data objects according to a classification network and according to a process hierarchy of steps and algorithms. For a more detailed description of generating a data network using a process hierarchy and a class network, see U.S. Patent No. 8,319,793, the contents of which are incorporated herein by reference. Each digital image comprises pixel values associated with the locations of each of the pixels 19. The image analysis program operates on the digital pixel values and links the pixels to form objects. Each object is linked to a set of pixel locations based on the associated pixel values. For example, an object is generated by linking to the object those pixels having similar characteristics, such as hue, saturation and brightness as defined by the pixel values. Alternatively, the pixel values can be expressed in a 3-value color space. For example, in the RGB color space, three 3-digit numbers in the range from zero to 255 define the color. The three numbers represent the amounts of red, green and blue in the represented color. For example, red is represented as 255-0-0, dark green is represented as 0-100-0, royal blue is designated as 65-105-225, white is represented as 255-255-255, and black is represented as 0-0-0. Smaller numbers represent darker colors, so 100-100-100 is a darker gray than 200-200-200, and 0-0-128 is a darker blue (navy) than straight blue 0-0-255. Although the operation of system 10 is described in relation to the RGB color space, other color spaces and representations may also be used, such as the CMYK (cyan, magenta, yellow, black) color model, the CIE 1931 color space, the 1964 xyz color space or the HSV and HSL representation of the RGB color space. Thresholds of brightness at pixel locations that are grouped together can be obtained from a histogram of the pixel values in the digital image. The pixels form the lowest hierarchical level of hierarchical network 16.

In one example, pixels having the color and intensity imparted by the biomarker stain are identified and linked to first objects 17. The first objects 17 form the second hierarchical level of hierarchical network 16. Then data objects are linked together into classes according to membership functions of the classes defined in the class network. For example, objects representing nuclei are linked together to form objects 20-21 in a third hierarchical level of hierarchical network 16. In FIG. 2, some of the first objects 17 correspond to stained pixels of the nuclear membrane of a nucleus corresponding to object 20. In addition, another of the first objects 17 corresponds to stained pixels on the nuclear membrane of a separate nucleus represented by object 21. An additional object 22 is generated in a fourth hierarchical level of hierarchical network 16 and is linked to all of the objects that represent stained nuclei. Thus, the objects 20-21 corresponding to stained nuclei are linked to object 22.

The knowledge and the program flow of the image analysis program are separated in the software structure. The parameters by which the image analysis is performed, for example thresholds of size or brightness, can be changed without having to revise the process hierarchy of software steps. The image analysis software displays both the original digital images 11 as well as the corresponding processed images and heat maps on the graphical user interface 14. Pixels corresponding to classified and segmented objects in the digital images are colored, marked or highlighted to correspond to their classification. For example, the pixels of objects that are members of the same class are depicted in the same color.

The image analysis program is used to determine how cancer drugs (groups of molecules) are interacting with cancer cells in tissue samples taken from a cancer patient and thereby how the drug treatment is progressing. In an immunotherapy-related application, the image analysis program is used to quantify the statistical distribution of distances of specific immune cells (T-cells, B-cells, M1 and M2 macrophages) from tumor cells. Second, the program is used to measure the quantitative expression of individual antigens (e.g., HER2, VEGFR, PD-L1) using image analysis of immunohistochemical stained slides. In particular, the staining intensity in the membrane of the tumor cells is measured. Third, the program is used to measure the statistics of distances of immune cells to specific (antigen) positive stained tumor cells. This statistical information is displayed on the user interface 14.

Immune cells are capable of killing malignant cancer cells if the cancer cells are recognized as having characteristic tumor antigens on their surfaces. In present therapeutic approaches, a single tumor antigen is used to guide the immune cells to their targets. Targeting cancer cells based on a single antigen, however, causes severe side effects for cancer patients because the targeted tumor antigen is sometimes present in healthy cells as well, and the cancer drugs cause these healthy cells to be attacked as well. The cancer cells can be more selectively attacked by guiding immune cells to targeted cells that possess a plurality of predetermined antigens. A novel drug therapy employs a logical AND combination on targeted cells to guide the immune cells only towards those tumors cells on which at least two predetermined tumor antigens are simultaneously present on the cell surface of the tumor cell. The image analysis program analyzes tissue samples of a patient to determine whether the combination of two predetermined antigens is present on individual cells. For example, the image analysis program determines whether a significant number of regions having the color of a first antigen stain are in close proximity on cell membranes to regions having the color of a second antigen stain. The cells identified as having both antigens have a higher probability of being tumor cells than cells identified as having only a single predetermined antigen.

If the image analysis determines that a large number of cells in the tissue sample have been identified as cancer cells because they possess the two predetermined antigens, then a series of three drugs (groups of molecules) are administered to the patient. The first drug is an AND molecule, the second drug is a glue molecule, and the third drug is a binding molecule that activates an immune response.

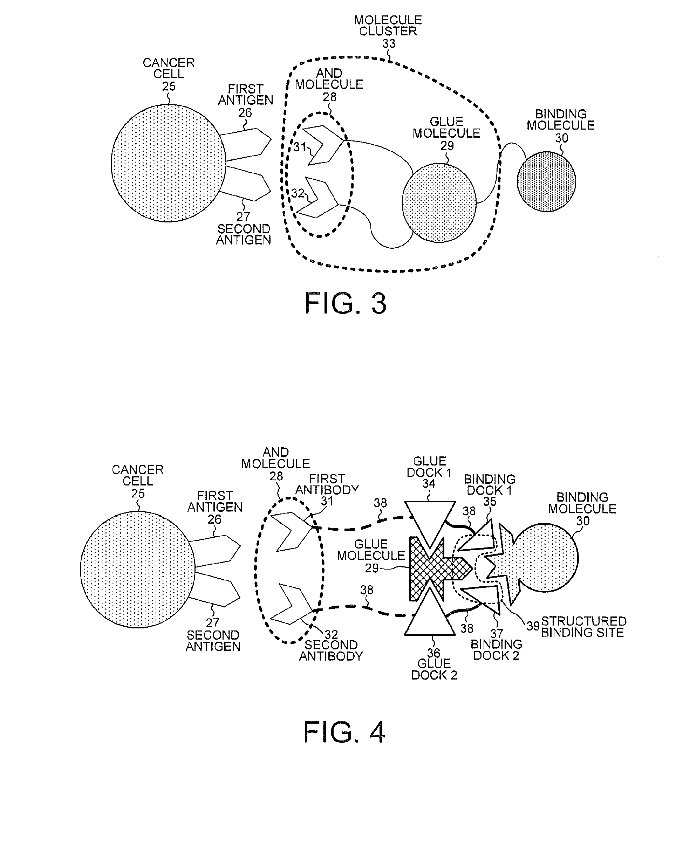

FIG. 3 illustrates how the three drugs are administered. FIG. 3 shows a cancer cell 25 with a first antigen 26 and a second antigen 27. The antigens 26-27 are specifically chosen to be those that are most likely to be present on cancerous cells. FIG. 3 also shows the AND molecule 28, the glue molecule 29 and the binding molecule 30 (also called the immune activating molecule).

A tissue sample of the patient is stained with two biomarkers. The first biomarker is an antibody with an attached first-colored stain that binds to a first tumor antigen, and the second biomarker is another antibody with an attached second-colored stain that binds to a second tumor antigen. Cancer cells are determined to be present in the patient's tissue sample if image analysis recognizes that both colors are present together on the membranes of cells.

If the patient's tissue sample is determined to have cancer cells with the two predetermined tumor antigens, then the first drug, the AND molecule 28, is administered to the patient. The AND molecule 28 has two antigen-specific antibodies 31-32 that are capable of binding to the first tumor antigen 26 and the second tumor antigen 27, respectively. The AND molecule 28 also includes other molecules (not shown in FIG. 3) that are attached to the antibodies 31-32. After allowing a first predetermined interval to elapse, the second drug, glue molecule 29, is administered. In one implementation, the first predetermined interval is one day. Glue molecule 29 binds to the two antibodies 31-32 wherever these two antibodies are in close proximity. The AND molecule 28 attached to the glue molecule 29 forms a molecule cluster 33 that includes new structured binding sites (epitopes).

A second predetermined interval is allowed to elapse. In one implementation, the second predetermined interval is several hours. In one embodiment, glue molecule 29 is designed such that any binding to a single antibody is too weak to be persistent, and glue molecule 29 separates from any single antibody due to thermodynamics within a time period shorter than the second interval. However, if glue molecule 29 binds to both antibodies 26-27 at the same time, much more energy is required to break both bonds to separate glue molecule 29 from AND molecule 28, and the molecules stay bound together longer than the second interval so that the third drug binds only to molecule cluster 33. Alternatively, glue molecule 29 can be designed so that a double binding between AND molecule 28 and glue molecule 29 causes a conformational change (a folding structure) of the glue molecule that creates a binding site for the binding molecule 30. Glue molecules 29 that do not bind to AND molecules 28 are metabolized or are passed from the patient's body. After the second predetermined interval has elapsed, the third drug, binding molecule 30, is administered. Binding molecule 30 (an immune activating molecule) binds only to the newly formed binding site on the glued molecule cluster 33. Binding molecule 30 attracts immune cells that subsequently kill the cancer cell 25.

In another embodiment, glue molecule 29 and binding molecule 30 (the immune activating molecule) are a single molecule that has two different structures formed by a conformational change. In this case, glue molecule 29 undergoes a conformational change (e.g., by folding) when glue molecule 29 binds to AND molecule 28 that creates a binding site for immune cells instead of a binding site for the binding molecule 30. In this embodiment, a separate binding molecule 30 is not used.

FIG. 4 is a schematic diagram illustrating a specific application of the therapy using the first medication 28, the second medication 29, and the third medication 30. The therapy is used to treat breast cancer in which image analysis determines that tissue samples are positively stained for both of the antigens Human Epidermal growth factor Receptor 2 (Her2) and PD-L1 (Programmed Death-Ligand 1). Her2 is a receptor tyrosine kinase on the surface of most cells of the human body. HER2 contributes to multiple protein pathways and enable cell survival and proliferation. The amplification of Her2, however, also promotes the proliferation of various tumor cells.

In a first step, the first medication 28 is administered to the patient. The first medication 28 includes the first antibody anti-HER2 31 and the second antibody anti-PD-1 32. Each of the antibodies 31-32 has a binding site for second medication 29 and another binding site for third medication 30. Glue dock 34 is the binding site of first antibody 31 for second medication 29, and binding dock 35 is the binding site of first antibody 31 for third medication 30. Glue dock 36 is the binding site of second antibody 32 for second medication 29, and binding dock 37 is the binding site of second antibody 32 for third medication 30. The binding sites are attached to first medication 28 by linker chains 38. In the patient's body, the first antibody 31 binds to the first antigen HER2, and the second antibody 32 binds to the second antigen Programmed Death-Ligand 1 (PD-L1) 27 (also known as the cluster of differentiation 274 (CD274)). PD-L1 is a checkpoint inhibitor and allows cells to escape from an immune response. Some types of cancer have acquired the antigen PD-L1 and use it as a tumor escape mechanism. The logical AND therapy uses the PD-L1 antigen together with a second antigen to recognize these tumor cells.

In the second step, the second medication 29 is administered to the patient. The second medication 29 is the glue molecule that attaches to the first medication 28 only if both glue dock 34 and glue dock 36 bind to second medication 29, which can occur only if the glue docks 34 and 36 are in the vicinity of one another. The docks 34, 36 are in the vicinity of one another if they are separated from one another by no more than the maximum dimension of a large biological molecule. Thus, glue dock 34 and glue dock 36 are in the vicinity of one another if first antibody 31 has bound to first antigen 26, second antibody 32 has bound to second antigen 27, and both first and second antigens 26-27 are located close to one another on the membrane of a cell. When glue docks 34 and 36 bind to glue molecule 29, a structured binding site 39 is formed for the third medication (the immune-activating molecule) 30.

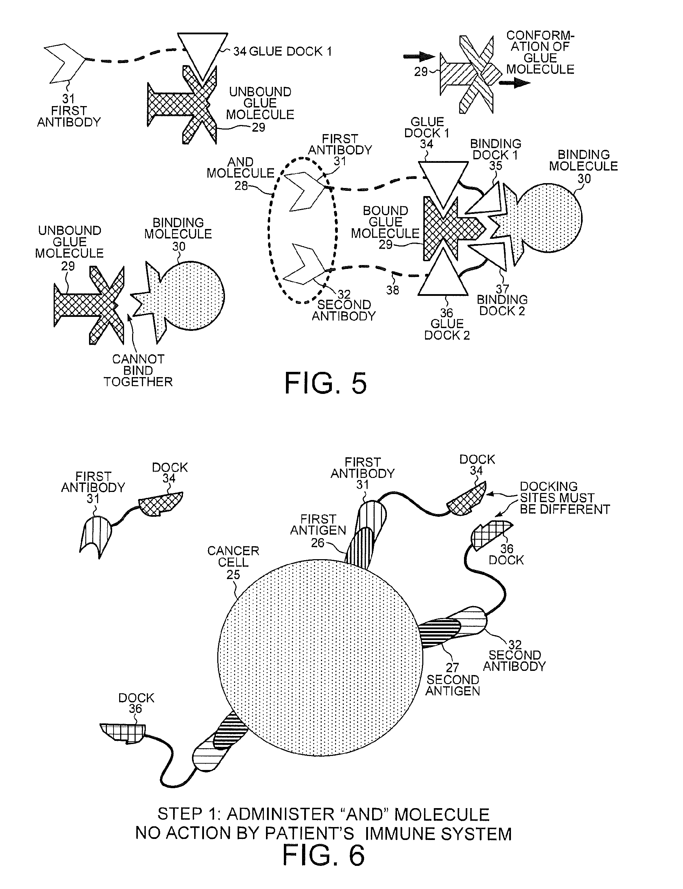

In the third step, the third medication 30 is administered. The third medication 30 is an immune activating molecule that specifically binds to the structure of the binding site 39 formed by the second medication 29 and the binding docks 35 and 37 of the first medication 28. The selective binding of the third medication 30 to the second medication 29 can be improved by designing the second medication 29 to change its structure when bound to both of the glue docks 34 and 36. FIG. 5 illustrates a bound configuration of second medication 29 that permits the structured binding site 39 to be formed when the second medication 29 is bound to the glue docks 34 and 36 of both antibodies 31-32. FIG. 5 also illustrates an unbound configuration of the second medication 29 that is present at all times when the second medication is not simultaneously bound to both antibodies 31-32, such as when the second medication is bound to only one of the antibodies 31-32. The third medication 30 is unable to attach to the unbound configuration of the second medication 29 because the structured binding site 39 cannot be formed from the unbound configuration. FIG. 5 also illustrates a schematic representation of the binding-induced conformational change that occurs in second medication 29 when it binds to both of the glue docks 34 and 36. The conformational change forms the epitope 39 (binding site).

Another specific application of the therapy using the first medication 28, the second medication 29, and the third medication 30 involves the treatment of malignant melanoma with anti-VEGF and anti-cMET as the antibodies that bind to the VEGF and cMET antigens on the membranes of tumor cell.

FIG. 6 illustrates another application using the first medication 28, the second medication 29, and the third medication 30. In the therapy shown in FIG. 6, the first medication 28 is a combination of two unbound molecules. The first molecule includes first antibody 31 linked to dock 34, and the second molecule includes second antibody 32 linked to dock 36. Although the first and second molecules typically include naturally occurring antibodies, such as anti-HER2 and anti-PD1, artificially designed molecules can also be used that have binding sites to the predetermined antigens used to identify tumor cells, such as HER2 and PD-L1 or VEGF and cMET. The two molecules need not be linked to one another at the time they are administered to the patient in the first step. The two molecules can be administered together or one after the other. The delay between administering the two molecules should be much shorter than the first predetermined interval. At locations on the surfaces of cancer cells at which both first antigen 26 and second antigen 27 are in close proximity, the first and second molecules bind to the antigens such that the linked docks 34 and 36 are able to contact each other. But after the administration of the first medication 28 in the first step, there is no action by the patient's immune system.

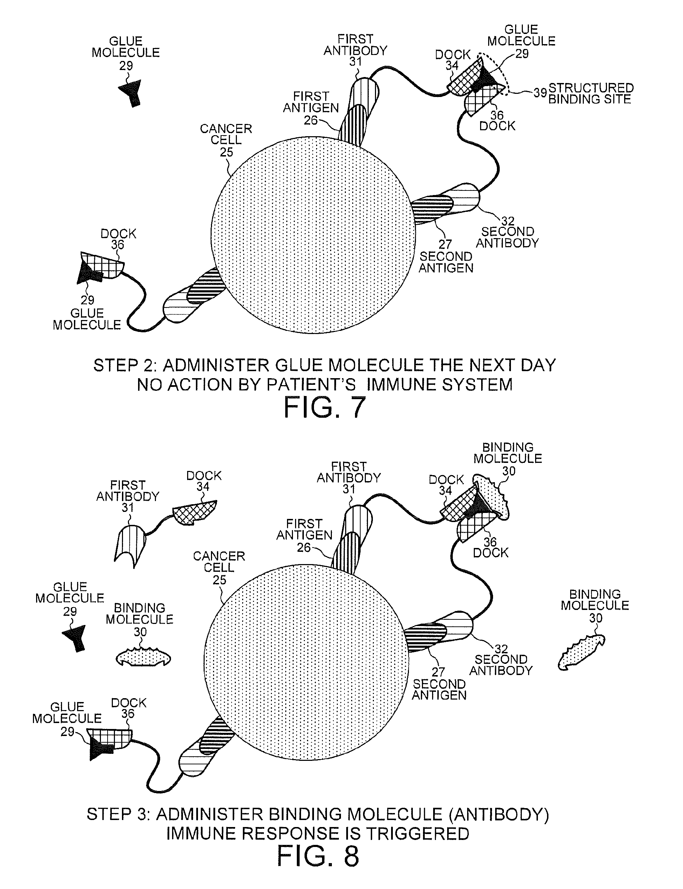

FIG. 7 illustrates the second step in which the second medication 29 is administered to the patient. In this application, the second medication 29 is administered to the cancer patient the day after the first medication 28 is given. The second medication 29 is the glue molecule that can attach to either dock 34 or dock 36 or both docks 34 and 36. The docking sites of dock 34 and dock 36 are different from one another. FIG. 7 shows a glue molecule 29 that is unbound, another glue molecule that is bound only to a dock 36 and a third glue molecule 29 that is bound to both a dock 34 and a dock 36. But a structured binding site 39 is formed only if the second medication is bound both to dock 34 and to dock 36. In this application, glue molecule 29 binds tightly to the docks 34 and 36 and does not dislodge due to thermodynamics, as in the first application. The glue molecule 29 binds to one dock and then waits until a second dock is present to form the structure binding site 39. Even after structured binding sites are formed on molecules attached to cancer cells, however, no immune response it triggered. Although the structure binding site 39 is shown in FIG. 7 as including parts of dock 34, dock 36 and the glue molecule 29, in other implementations the glue molecule 29 does not form part of the binding site 39, but rather just binds dock 34 to dock 36.

FIG. 8 illustrates the third step in which the third medication 30 is administered. The third medication 30 selectively binds only to the structured binding sites 39 formed by the combination of the second medication 29 with the docks 34 and 36 of the first medication 28. The third medication triggers a response from the patient's immune system by attaching to the binding sites 39 and then attracting immune cells to the cancer cell that has been identified by the two antigens 26-27. Third medication 30 can have a binding site for an immune cell or can trigger an aggressive response from any immune cell in its vicinity.

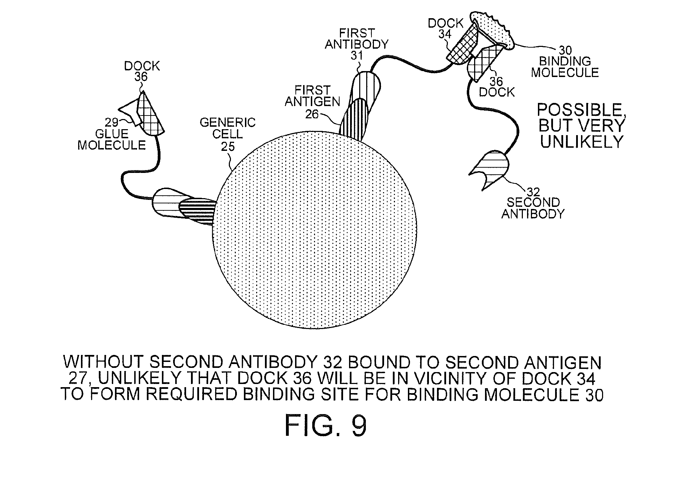

FIG. 9 illustrates a rare occurrence in which the third medication 30 attracts immune cells to a cell that does not have both antigens 26-27 in close proximity on the cell membrane. This is an example of a harmful side effect of typical cancer medications that also harm cells that are not cancerous. In the situation depicted in FIG. 9, the docks 34 and 36 of both of the unbound molecules of the first medication 28 happen to have come in contact with each other at the precise time that a glue molecule 29 was also present, thereby creating a binding site 39 for the third medication 30. This causes immune cells to attack a generic cell 25 that is not cancerous. In the vast majority of cases, however, the docks 34 and 36 of the unbound molecules would come in contact with each other only if the antibodies 31-32 are bound to antigens 26-27 in close proximity on a cell's surface.

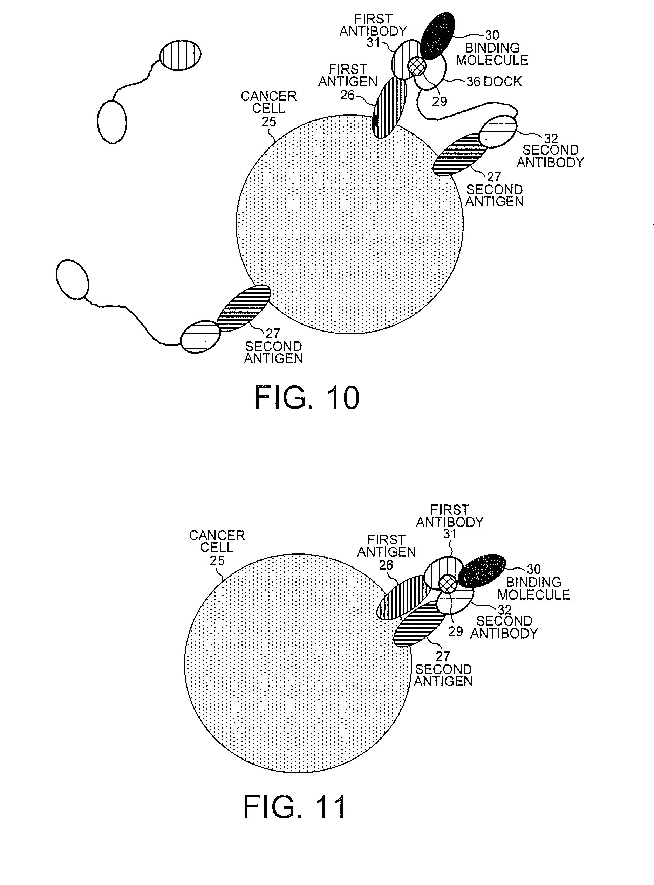

FIG. 10 illustrates a therapy in which the first medication 28 is a combination of a first molecule without a glue dock and a second molecule with a glue dock. The first molecule includes just the first antibody 31 without the glue dock 34. The first antibody 31 is chosen or designed to have a binding pocket for binding molecule 30. The second molecule includes second antibody 32 linked to dock 36. The second medication 29 binds the first antibody 31 to the dock 36 of the second antibody 32. The structured binding site 39 is then formed by portions of the first antibody 31 and the dock 36.

FIG. 11 illustrates another therapy in which neither of the molecules of the first medication 28 has a glue dock. Both of the antibodies 31-32 have a binding site for binding molecule 30. The second medication 29 binds the first antibody 31 to the second antibody 32. The structured binding site 39 is then formed by portions of the first antibody 31 and the second antibody 32.

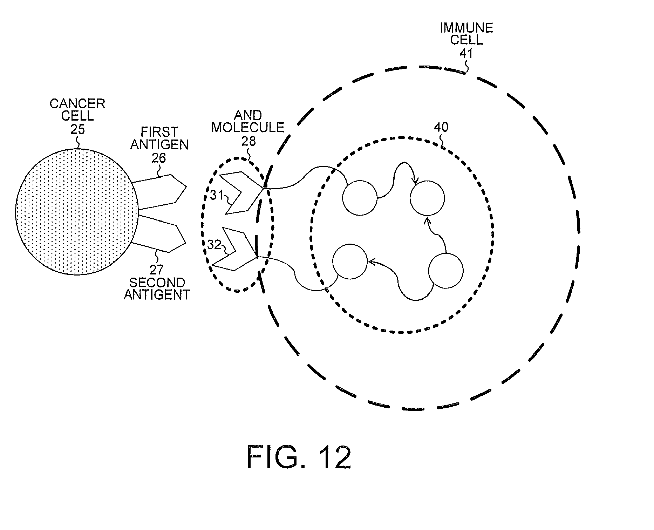

FIG. 12 illustrates a second approach in which selected immune cells are reprogrammed to perform the selective binding accomplished by AND molecule 28 and glue molecule 29. A part of the functional network 40 of an immune cell 41 is reprogrammed such that the immune cell approaches only those tumor cells on which the two antigens 26-27 are simultaneously present on the cell's surface. One way to reprogram immune cells is to extract selected immune cells from the patient's body and then to apply siRNA or miRNA on the extracted T-cells. Applying siRNA or miRNA on the extracted T-cells causes the down-regulation of certain proteins. The modified T-cells are then injected back into the patient. Moreover, the tumor antigens 26-27 are presented to the extracted dendritic cells. The pathway in the immune cell, including the different molecules binding to the tumor antigens, is reprogrammed in a way that represents the AND function of the AND molecule 28.

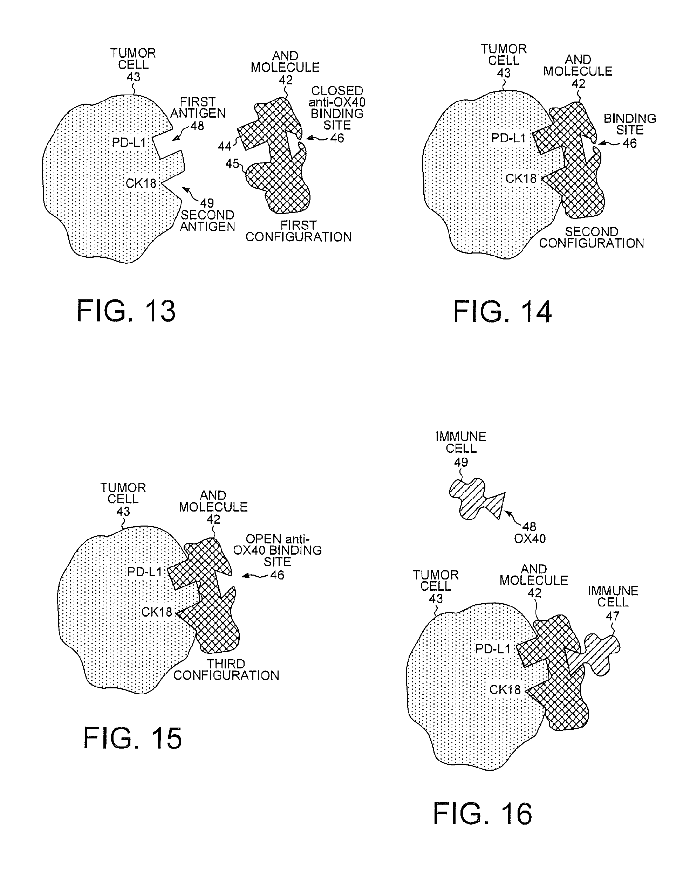

FIGS. 13-16 illustrate a single step therapy embodiment that uses an AND molecule 42 with three ligands to amplify an immune attack against tumor cells 43. The therapy using the triple-ligand AND molecule is based on conformational changes that occur in antibodies when they bind to antigens. In one embodiment, the therapy is used to treat prostate cancer in which image analysis has determined that tissue samples are positively stained for both of the antigens Programmed Death-Ligand 1 (PD-L1) and cytokeratin 18 (CK18) on the membranes of the tumor cells. For example, anti-CK18 is a protein-specific monoclonal antibody that can be used together with an attached dye to form a biomarker. Luminal epithelial cells of prostate glands are stained by anti-CK18. In humans, anti-CK18 is encoded by the gene KRT18/PIG46/CYK18. Patients for whom the single step therapy is appropriate are identified by staining their tissue samples with antibody biomarkers that bind to the antigens PD-L1 and CK18. The therapy is likely to be effective if the stained colors of both the biomarkers PD-L1 and CK18 are present on the membranes of individual cells having a predetermined structure in a patient's tissue sample.

FIG. 13 illustrates a novel AND molecule 42 being just administered to a patient as the active ingredient in a medication. AND molecule 42 is synthesized to include the first antibody anti-PD-1 44 and the second antibody anti-CK18 45. In one implementation, the first antibody is linked to the second antibody by a linker chain in the AND molecule. AND molecule 42 also has a binding site 46 for an immune cell, such as a T cell. In one embodiment, binding site 46 is anti-OX40 (anti-DE134 or OX40-Ligand), which binds to the tumor necrosis factor (TNF) receptor OX40 (CD134) on immune T cells. AND molecule 42 has three configurations.

When the medication is first administrated to the patient, AND molecule 42 has the first configuration as schematically shown in FIG. 13. In the first configuration, the binding site 46 for immune cells is closed. In the first configuration, the first antibody 44 has a shape that binds to a first antigen PD-L1 48 on the outside of the membrane of tumor cell 43. The binding compatibility is illustrated by both the first antibody 44 and the first antigen 48 having square shapes. In the first configuration, however, the shape of the second antibody anti-CK18 45 cannot bind to a second antigen cytokeratin 18 (CK18) 49 on the surface of tumor cell 43. For example, the second antibody cannot bind to its associated antigen because the antibody is in a closed state in the first configuration. The binding incompatibility is illustrated by the second antibody 45 having a rounded shape that cannot fit into the triangular shape of the second antigen 49. Thus, the first configuration of AND molecule 42 cannot bind to a cell that exhibits only the second antigen CK 18 without having the first antigen PD-L1 48 in the vicinity. This characteristic prevents AND molecule 42 from binding to cells that are probably not cancerous because they do not have both the first and second antigens on their membranes. For example, first antigen PD-L1 48 is a checkpoint inhibitor that allows cells to escape from an immune response and is likely present on some types of tumor cells. Cells with only the second antigen CK18 49 but without the first antigen PD-L1 48 are less likely to be malignant.

FIG. 14 illustrates the second configuration of AND molecule 42. After the first antibody 44 on AND molecule 42 binds to the first antigen 48 on tumor cell 43, AND molecule 42 undergoes a first conformation in which the shape of the second antibody anti-CK18 45 changes to fit the binding location of the second antigen CK18 49 on the surface of tumor cell 43. The binding compatibility between second antibody 45 and second antigen 49 is illustrated in FIG. 14 by both having triangular shapes. After the first conformation that forms the second configuration of the first medication, AND module 42 is more tightly bound to tumor cell 43.

FIG. 15 illustrates a third configuration of AND molecule 42 that occurs after a second binding-induced conformation. The second conformation results when AND molecule 42 bends so that both the first and second antibodies 44-45 are bound to the surface of tumor cell 43. In the third configuration of AND molecule 42, binding site 46 for the receptor OX40 on T cells is opened. After the second conformation, AND molecule 42 is configured into a triple-ligand state.

FIG. 16 illustrates an immune cell 47 binding to AND molecule 42. The receptor OX40 48 on T cells specifically binds to the structure of the binding site 46 opened by the second conformation of AND molecule 42. FIG. 16 illustrates the receptor OX40 48 of T cell 47 bound to the anti-OX40 binding site 36 of AND molecule 42, which in turn is bound to tumor cell 43. Immune cell 47 becomes aggressive when bound to binding site 46 and attacks tumor cell 43. FIG. 16 also illustrates an immune cell 49 with an unbound receptor OX40 48. Unbound immune cell 49 is unable to attach to the closed binding site 46 of the first and second configurations of AND molecule 42. Thus, immune cells do not bind to AND molecules that are not yet bound to cells exhibiting both the first and second antigens 48-49, which are characteristic of tumor cells.

Although the present invention has been described in connection with certain specific embodiments for instructional purposes, the present invention is not limited thereto. Accordingly, various modifications, adaptations, and combinations of various features of the described embodiments can be practiced without departing from the scope of the invention as set forth in the claims.

## Claims

1. A method comprising:
administering a first medication to a patient, wherein the first medication includes a first antibody and a second antibody, wherein the first antibody is linked to a first dock, and the second antibody is linked to a second dock;
after the elapse of a first predetermined interval, administering a second medication to the patient, wherein the second medication forms a structured binding site when the second medication is simultaneously bound to both the first dock and the second dock; and
after the elapse of a second predetermined interval, administreing a third medication to the patient, wherein the third medication binds only to the structured binding site and activates immune cells of the patient.

2. The method of claim 1, wherein the patient has breast cancer.

3. The method of claim 1, wherein the first antibody is anti-HER2, and the second antibody is anti-PD-L1.

4. The method of claim 1, wherein the first antibody is linked to the first dock by a linker chain.

5. The method of claim 1, wherein the first predetermined interval is one day.

6. A method comprising:
administering a first medication to a patient, wherein the first medication includes a first molecule and a second molecule, wherein the first molecule includes a first antibody and a first dock, and wherein the second molecule includes a second antibody and a second dock;
after the elapse of a first predetermined interval, administering a glue molecule to the patient, wherein the glue molecule binds to both the first dock and the second dock, and wherein a structured binding site is formed when the glue molecule is simultaneously bound to both the first dock and the second dock; and
after the elapse of a second predetermined interval, administreing a binding molecule to the patient, wherein the binding molecule binds only to the structured binding site and activates immune cells of the patient.

7. The method of claim 6, wherein the patient has breast cancer.

8. The method of claim 6, wherein the first antibody is anti-HER2, and the second antibody is anti-PD-L1.

9. The method of claim 6, wherein the first antibody is anti-PD-L1, and the second antibody is anti-CK18.

10. The method of claim 6, wherein the first predetermined interval is one day.

11. The method of claim 6, wherein the second molecule is administered to the patient after the first molecule.

12. A method comprising:
generating image objects from a digital image of tissue from a cancer patient;
determining that the image objects are cells that have been stained with both a first biomarker and a second biomarker; and
administering an AND molecule to the cancer patient, wherein the AND molecule includes a first antibody and a second antibody, wherein the second antibody has a shape that can bind to its corresponding antigen only when the first antibody is bound to its corresponding antigen, and wherein the AND molecule includes a binding site that can bind to immune cells only when both the first antibody and the second antibody are bound to their corresponding antigens.

13. The method of claim 12, wherein the first antibody is anti-Her2, and the second antibody is anti-PD-L1.

14. The method of claim 12, wherein the first biomarker is anti-Programmed Death-Ligand 1 (anti-PD-L1), and the second biomarker is anti-cytokeratin 18 (anti-CK18).

15. The method of claim 14, wherein the patient has prostate cancer.

16. The method of claim 12, wherein the first biomarker is anti-Human Epidermal growth factor Receptor 2 (anti-Her2), and the second biomarker is anti-Programmed Death-Ligand 1 (anti-PD-L1).

17. The method of claim 12, wherein the second biomarker stains luminal epithelial cells.

18. The method of claim 12, wherein the binding site is anti-OX40 that binds to tumor necrosis factor receptor OX40 on immune cells.

19. The method of claim 12, wherein the binding site is opened by a conformation of the AND molecule induced by the binding of both the first antibody and the second antibody to their corresponding antigens.
